# EUROPEAN PATENT APPLICATION

(11) **EP 4 350 003 A1**
(43) Date of publication of application: **10.04.2024**
(21) Application number: 22382944.1
(22) Date of filing: 06.10.2022
(51) Int. Cl.: C12Q 1/6841

(54) **LOW INPUT CHROMOSOME CONFORMATION CAPTURE METHODS**

(71) Applicant: Fundació Institut de Recerca Contra la Leucèmia Josep Carreras, 08916 Barcelona (ES); Fundació Institut d'Investigació en Ciències de la Salut Germans Trias i Pujol (IGTP), 08916 Badalona (ES)
(72) Inventor: JAVIERRE MARTÍNEZ, Biola María, 08916 BADALONA (ES)
(74) Representative: ZBM Patents - Zea, Barlocci & Markvardsen

(57) **Abstract**

The present invention provides a method for identifying at least one nucleic acid sequence that interacts with at least one target nucleic acid sequence, the method comprising the steps of: (a) crosslinking a nucleic acid composition comprising the at least one target nucleic acid sequence; (b) fragmenting the crosslinked nucleic acid composition, thereby producing fragmented crosslinked nucleic acid sequences; (c) marking the ends of the nucleic acid sequences with an affinity tag;(d) ligating the nucleic acid sequences to produce nucleic acid sequences with ligation junctions comprising the affinity tag; (e) reversing the crosslinking; (f) shearing the nucleic acid sequences; (g) purifying the nucleic acid sequences with ligation junctions comprising the affinity tag by contacting with a ligand of the affinity tag; (h) A-tailing and amplifying the purified nucleic acid sequences; (i) isolating the amplified nucleic acid sequences that comprise the at least one target nucleic acid sequence or a portion of the at least one target nucleic acid sequence; and (j) sequencing the isolated nucleic acid sequences to identify nucleic acid sequences that interact with the at least one target nucleic sequence; with the proviso that the method does not comprise a step to remove the affinity tag from non-ligated ends.

## Description

### Technical Field

The present invention belongs to the field of methods for studying chromatin structure; in particular high-throughput methods for studying chromatin conformation in samples with low cell numbers. The methods of the invention are particularly useful for studying clinical samples or rare cell populations.

### Background Art

The study of three-dimensional genome architecture has been revolutionized by the introduction of 3C (chromosome conformation capture) techniques, in particular Hi-C (high throughput chromosome conformation capture), which is capable of identifying the entire ensemble of chromosomal interactions within a cell population.

Hi-C is based on proximity ligation, such that chromatin sequences that are in physical proximity are ligated to each other, purified, and then analyzed with next-generation sequencing technology. However, the enormous combinatorial complexity of sequence libraries generated in Hi-C does not allow a reliable and reproducible identification of interactions between specific individual regulatory regions-such as promoters or enhancers-in a rutinary manner since the extremely high costs of sequencing.

To circumvent these shortcomings, other techniques have been developed to interrogate the interactions between individual regions-for example, circular chromosome conformation capture (4C), or Promoter Capture Hi-C (PCHi-C). However, these techniques are costly and cumbersome or rely on the availability of millions of cells, typically ranging between 30-50 million cells per biological replicate, which makes them incompatible with clinical scenarios or for studying rare cell populations.

Thus, in spite the efforts made so far, there is still a need for simple Hi-C methods that allow the study of 3D chromatin at high resolution from low cell numbers, in particular in clinical samples.

### Summary of Invention

The present inventors have developed an optimized low input capture Hi-C method that allows studying the chromatin organization of samples with very low cell numbers in a cost-effective manner.

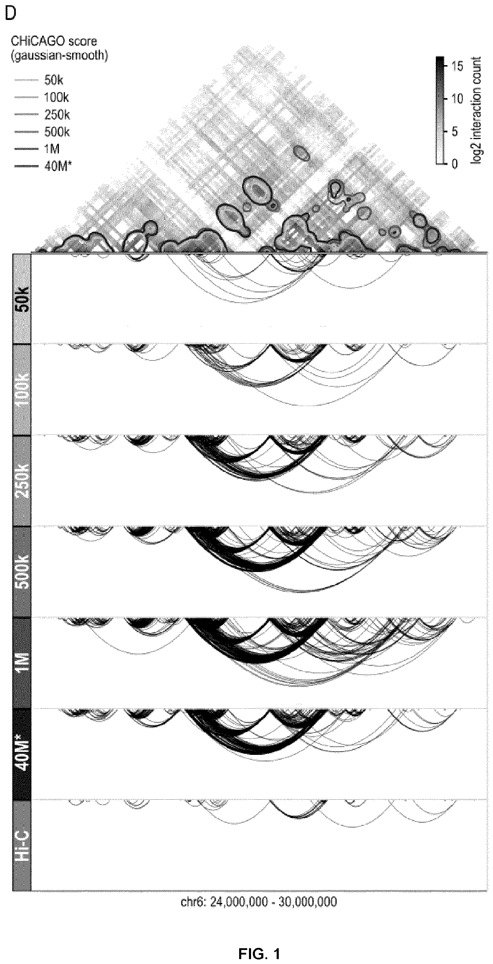

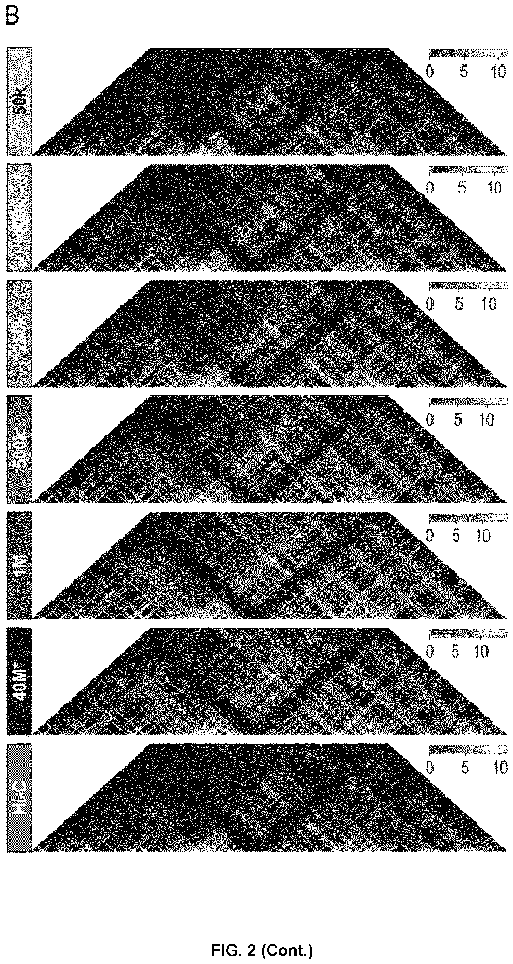

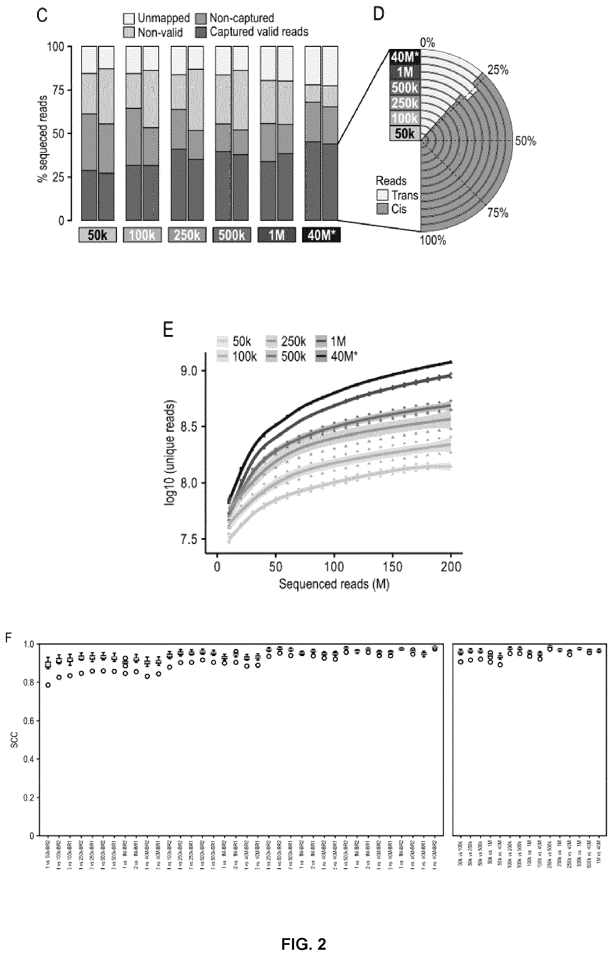

As shown in the examples below, through extensive experimentation the present inventors have surprisingly found that altering the order of several steps of Hi-C methods -for example, the steps after pulling-down the fragments containing labelled ligation junctions-and skipping other steps -for example, the step where biotin is removed from unligated ends-, the method allows for the interrogation of interactions between individual chromatin regions in samples derived from as low as 50,000 cells without significantly loosing valid sequence reads (see Figure 1D, Figure 2B and C below). Remarkably, the method of the invention was able to achieve a >10-fold enrichment of read pairs involving promoters when compared with Hi-C methods using 800 times less of the starting cells.

This was highly unexpected considering that it was well stablished in the prior art that for interrogating interactomes at high resolution it was necessary to remove biotin from non-ligated ends to improve sequencing efficiency or to use starting materials with cell numbers three orders of magnitude higher-typically 30-50 million cells.

Therefore, the method provided by the present inventors allows obtaining high quality interactomes for specific regions in a cost-effective manner and from low cell numbers, which will allow to expand the chromatin study to low abundance cell populations and offer to uncover novel factors and regulatory networks involved in disease pathogenesis.

Thus, in a first aspect, the invention provides a method for identifying at least one nucleic acid sequence that interacts with at least one target nucleic acid sequence, the method comprising the steps of: (a) crosslinking a nucleic acid composition comprising the at least one target nucleic acid sequence; (b) fragmenting the crosslinked nucleic acid composition, thereby producing fragmented crosslinked nucleic acid sequences; (c) marking the ends of the nucleic acid sequences with an affinity tag; (d) ligating the nucleic acid sequences to produce nucleic acid sequences with ligation junctions comprising the affinity tag; (e) reversing the crosslinking; (f) shearing the nucleic acid sequences; (g) purifying the nucleic acid sequences with ligation junctions comprising the affinity tag by contacting with a ligand of the affinity tag; (h) A-tailing and amplifying the purified nucleic acid sequences; (i) isolating the amplified nucleic acid sequences that comprise the at least one target nucleic acid sequence or a portion of the at least one target nucleic acid sequence; and (j) sequencing the isolated nucleic acid sequences to identify the at least one nucleic acid sequence that interacts with the at least one target nucleic sequence; with the proviso that the method does not comprise a step to remove the affinity tag from non-ligated ends.

Nucleic acid sequences that are identified to interact with target sequences are candidates to be regulatory elements that are required for proper genetic control. Their alteration may alter gene expression and contribute to disease, therefore linking these elements to their target genes could provide potential new drug targets for new therapies.

Thus, in a second aspect, the invention provides a method of identifying one or more interacting nucleic acid sequences that are indicative of a particular disease state, the method comprising (i) performing the method as defined in the first aspect on a nucleic acid composition sample obtained from an individual with a particular disease state; (ii) quantifying a frequency of interaction between at least one nucleic acid sequence and the at least one target nucleic acid sequence; and (c) comparing the frequency of interaction in the nucleic acid composition sample from the individual with said disease state with the frequency of interaction in the nucleic acid composition sample obtained from a healthy subject, such that a difference in the frequency of interaction in the nucleic acid composition is indicative of a particular disease state.

In a third aspect, the invention provides a kit of parts for identifying at least one nucleic acid sequence that interacts with at least one target nucleic acid sequence, the kit comprising buffers and reagents capable of performing the method as defined in the first aspect.

In a fourth aspect, the invention provides the use of the kit of parts according to the third aspect for identifying at least one nucleic acid sequence that interacts with at least one target nucleic acid sequence.

### Brief Description of Drawings

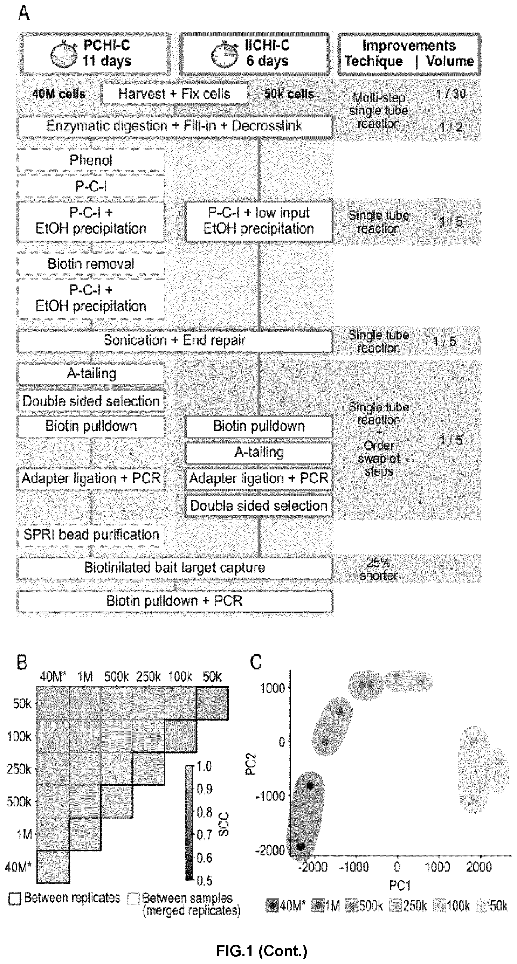

Fig. 1 shows: a. Schematic comparison of the PCHi-C vs liCHi-C workflow. Dash-bordered boxes denote removed steps from the original PCHi-C protocol. b. Heatmap displaying stratum adjusted correlation coefficient (SCC) between promoter interactomes of human naive B cells obtained by liCHi-C and PCHi-C (*) using different cell numbers. Reproducibility between biological replicates and between pairs of merged biological replicates are surrounded by a black and grey lines respectively. 1000 (k), million (M). c. Principal Component Analysis of CHiCAGO significant interactions (CHiCAGO scores > 5) of biological replicates detected by liCHi-C and PCHi-C (40M*) using different cell numbers. d. Top: Interaction matrix at 50kb resolution generated with PCHi-C and 40 million cells. Colored contour plot over the interaction matrix represents gaussian smoothing (alpha=1.2) of the significant CHICAGO interactions detected by liCHi-C data using different numbers of input cells. Bottom: Significant interactions (arcs) detected with liCHi-C and PCHi-C (40M*) and Hi-C using different numbers of input cells

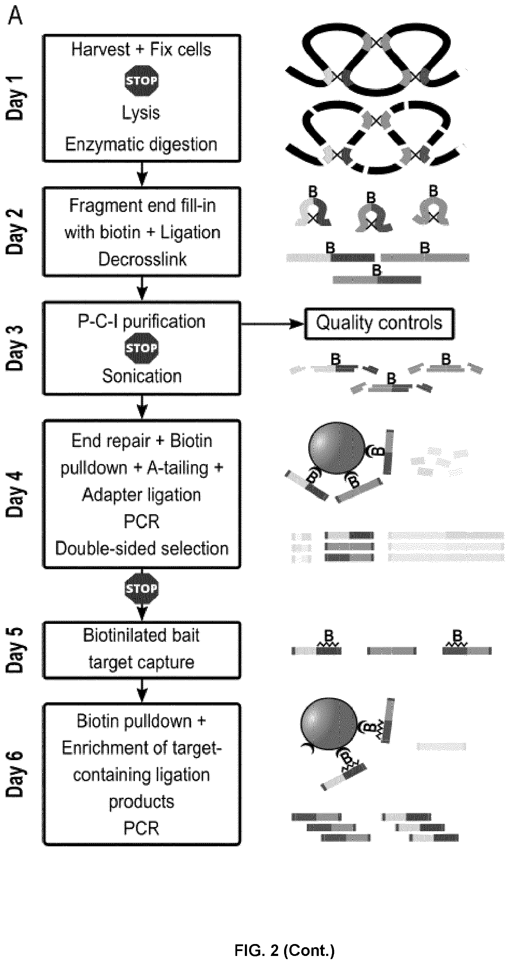

Fig. 2 shows: a. In-depth overview of the liCHi-C workflow. "STOP" icons symbolize points in which the protocol may be safely stopped up to several weeks by freezing the material. Optional quality controls of the first part of the protocol before sonicating may be performed at the expense of an extra day. b. Interaction matrices of liCHi-C, PCHi-C (40M*) and Hi-C binned at 50kb resolution of experiments with different numbers of input cells. 1000 (k), million (M). c. Proportions of reads passed through the different steps of HiCUP. d. Cis-trans interaction ratio of valid captured reads. Cis and trans mean interactions within and between chromosome respectively. e. Saturation plot representing unique reads respect to total number of sequenced reads. f. Boxplots of the stratum adjusted correlation coefficient (SCC) between promoter interactomes of human naive B cells obtained by PCHi-C (40M*) and liCHi-C using different cell numbers. Medians are represented by green lines. Each boxplot corresponds to the comparison between biological replicates (left) or merged replicates of experiments of different numbers of input cells (right).

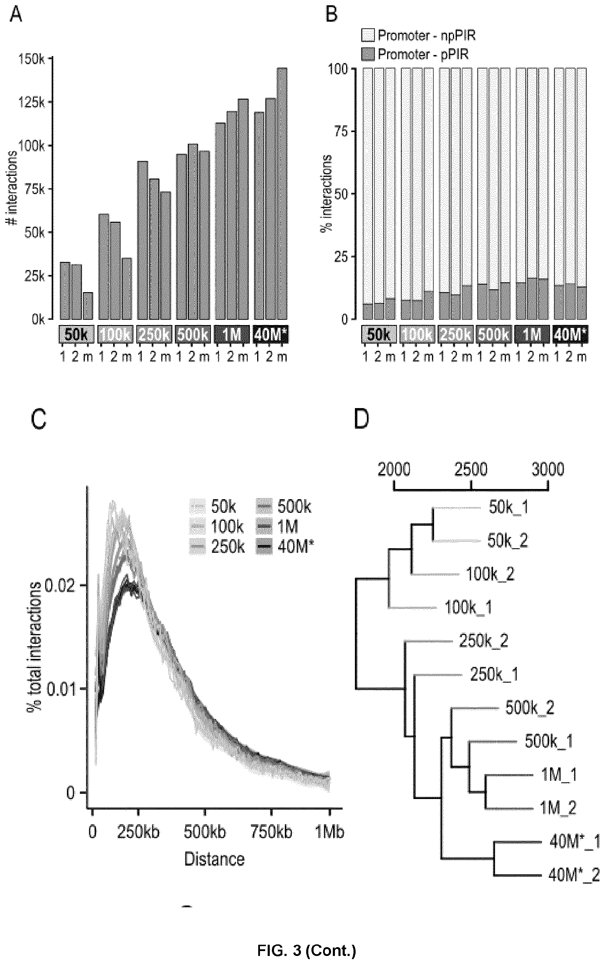

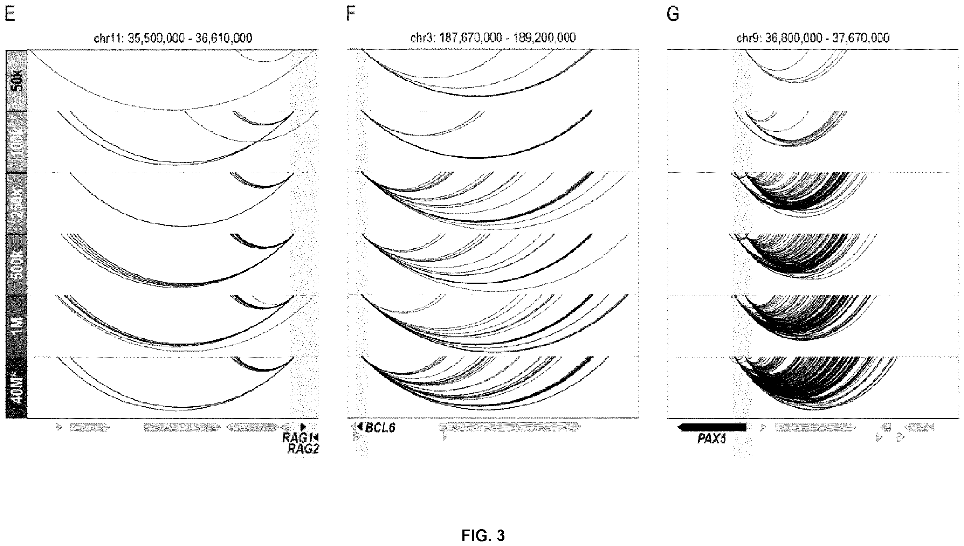

Fig.3 shows: a. Total number of CHiCAGO significant interactions (score >5) of biological replicates (1 and 2) and merged samples (m) obtained by PCHi-C (40M*) and liCHi-C using different numbers of naive B cells as input material. 1000 (k), million (M). b. Proportions of promoter-npPIR and promoter-pPIR of CHiCAGO significant interactions of biological replicates. c. Distance distribution of CHiCAGO significant interactions of merged samples. d. Dendrogram of hierarchical clustering with average linkage based on Euclidean distances of CHiCAGO significant interactions of biological replicates. RAG1/2 (e.) *BCL6* (f.) and *PAX5* (g.) promoter-centered interactions (arcs) according to liCHi-C data. Arrows symbolize gene placement and orientation along the genomic window.

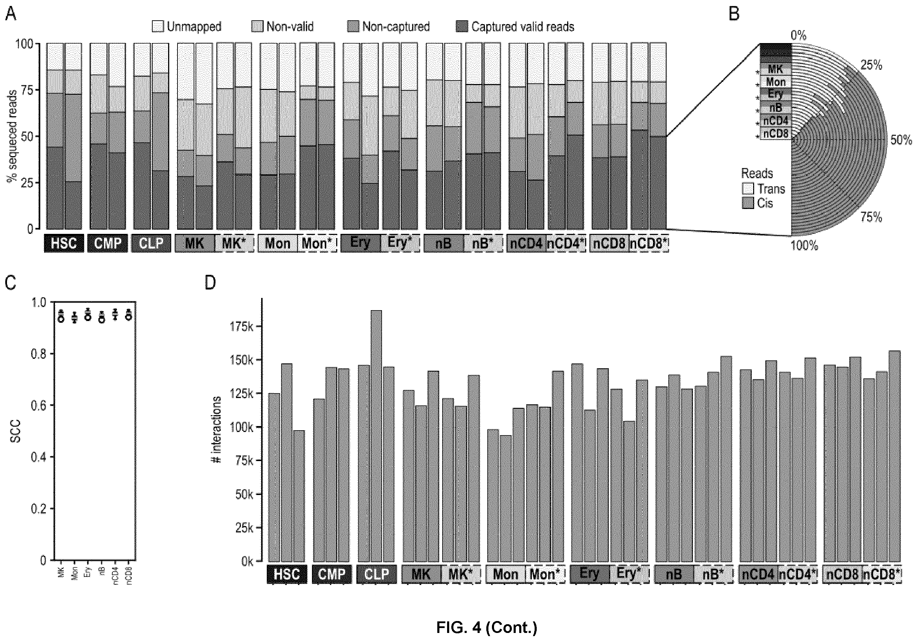

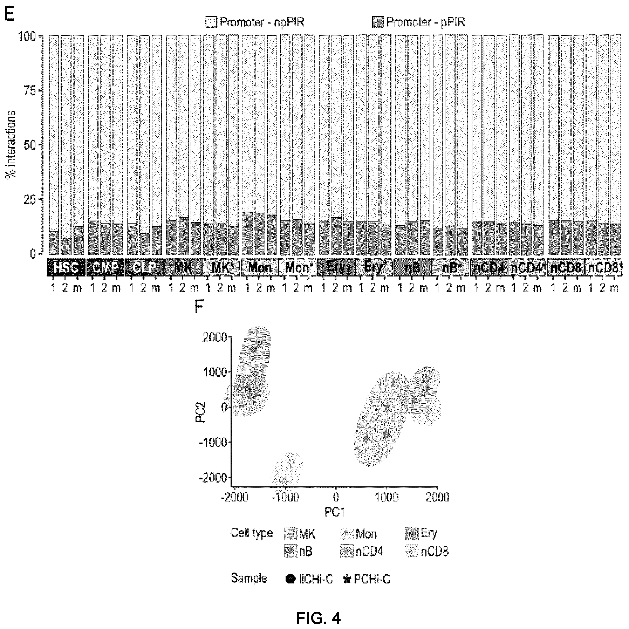

Fig. 4 shows: a. Proportions of liCHi-C reads passed through the different steps of HiCUP. Hematopoietic stem cell (HSC), common myeloid progenitor (CMP), common B cell lymphoid progenitor (CLP), megakaryocytes (MK), monocytes (Mon), erythroblast (Ery), naive B cell (nB), naive CD4+ cells (nCD4) and naive CD8+cells (nCD8). b. Cis-trans interaction ratio of liCHi-C valid captured reads. Cis and trans mean interactions within and between chromosome respectively. c. Boxplots of the stratum adjusted correlation coefficient (SCC) between promoter interactomes obtained by PCHi-C and liCHi-C. d. Total number of CHiCAGO significant interactions (score > 5) called in biological replicates (1 and 2) and merged samples (m). e. Proportions of promoter-npPIR and promoter-pPIR of significant interactions of biological replicates (1 and 2) and merged samples (m) detected by liCHi-C and PCHi-C. f. Principal component analysis of CHiCAGO

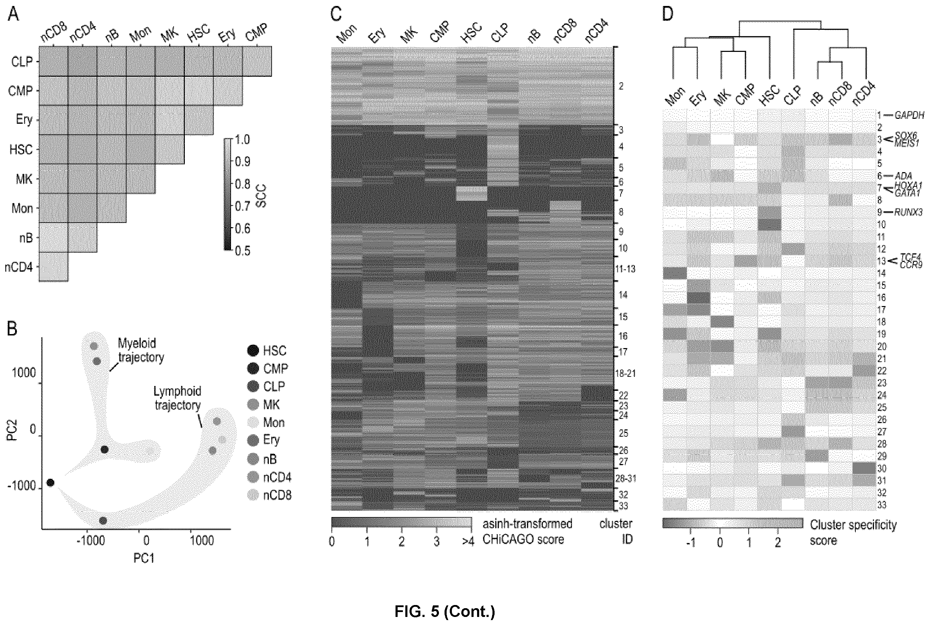

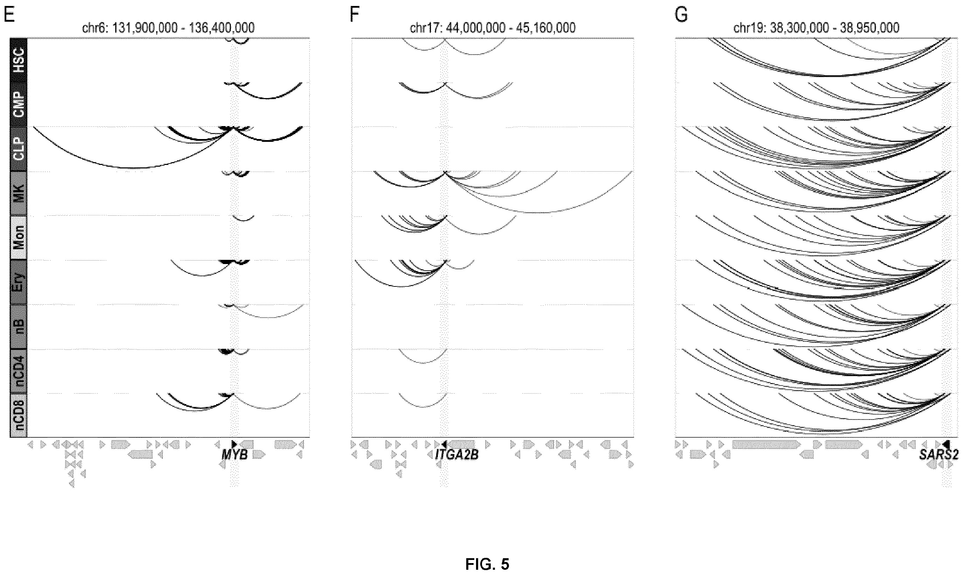

Fig. 5 shows: a. Heatmap displaying stratum adjusted correlation coefficient (SCC) between promoter interactomes of merged biological replicates. Hematopoietic stem cell (HSC), common myeloid progenitor (CMP), common B cell lymphoid progenitor (CLP), megakaryocytes (MK), monocytes (Mon), erythroblast (Ery), naive B cell (nB), naive CD4+ cells (nCD4) and naive CD8+ cells (nCD8). b. Principal Component Analysis of liCHi-C significant interactions (CHiCAGO scores > 5) from merged biological replicates. Shaded in grey are the predicted differentiation trajectories for both lymphoid and myeloid lineages. c. Heatmap of asinh-transformed CHiCAGO score of significant interactions in at least one cell type clustered using Autoclass algorithm. d. Top: Dendrogram of hierarchical clustering with average linkage based on Euclidean distances of CHiCAGO significant interactions of merged samples. Bottom: Heatmap of cluster specificity score of each Autoclass cluster. *MYB* (e.) *ITGA2B* (f.) and *SARS2* (g.) promoter-centered interactions (arcs) according to liCHi-data. Arrows symbolize gene placement and orientation along the genomic window. Shade depicts the gene promoter.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more."

Unless indicated otherwise, definite articles used herein, such as "the" also include the plural of the noun.

As above explained, in a first aspect the invention provides a method for identifying at least one nucleic acid sequence that interacts with at least one target nucleic acid sequence. The skilled in the art would understand that the method does not exclude additional steps before, after, or intercalated between the indicated steps.

The term "nucleic acid sequence" as used herein refers to any polymer of nucleotides (i.e., for example, adenine (A), thymidine (T), cytosine (C), guanosine (G), and/or uracil (U)). This polymer may or may not result in a functional genomic fragment or gene. A combination of nucleic acid sequences may ultimately comprise a chromosome. A nucleic acid sequence comprising deoxyribonucleosides is referred to as deoxyribonucleic acid (DNA). A nucleic acid sequence comprising ribonucleosides is referred to as ribonucleic acid (RNA).

It used herein, the term "target nucleic acid sequence" refers to the sequence of interest which is known to the user. Isolating only the ligated nucleic acid sequences which contain the target nucleic acid sequence or a portion of the target nucleic acid sequence allows to identify specific interactions with a particular sequence of interest.

The terms "interacts" or "interacting", refer to an association between two elements, for example in the present method, a genomic interaction between a nucleic acid sequence and a target nucleic acid sequence. The interaction usually causes one interacting element to have an effect upon the other, for example, silencing or activating the element it binds to. The interaction may occur between two nucleic acid sequences that are located close together or far apart on the linear genome sequence.

The term "nucleic acid composition", refers to any composition comprising nucleic acids. The nucleic acids within the nucleic acid composition may be organized into chromosomes, wherein the proteins (i.e., for example, histones) may become associated with the chromosomes having a regulatory function.

In an embodiment of the first aspect, the method comprises, in the following order, and optionally consecutively, the steps of: (a) crosslinking a nucleic acid composition comprising the at least one target nucleic acid sequence; (b) fragmenting the crosslinked nucleic acid composition, thereby producing fragmented crosslinked nucleic acid sequences; (c) marking the ends of the nucleic acid sequences with an affinity tag; (d) ligating the nucleic acid sequences to produce nucleic acid sequences with ligation junctions comprising the affinity tag; (e) reversing the crosslinking; (f) shearing the nucleic acid sequences; (g) purifying the nucleic acid sequences with ligation junctions comprising the affinity tag by contacting with a ligand of the affinity tag; (h) A-tailing and amplifying the purified nucleic acid sequences; (i) isolating the amplified nucleic acid sequences that comprise the at least one target nucleic acid sequence or a portion of the at least one target nucleic acid sequence; and (j) sequencing the isolated nucleic acid sequences to identify nucleic acid sequences that interact with the at least one target nucleic sequence; wherein the ligand of the affinity tag in step (g) is attached to a substrate and step (h) is carried out on the substrate.

In an embodiment of the first aspect, the method comprises the steps of: (a) crosslinking a nucleic acid composition comprising the at least one target nucleic acid sequence; (b) fragmenting the crosslinked nucleic acid composition, thereby producing fragmented crosslinked nucleic acid sequences; (c) marking the ends of the fragmented crosslinked nucleic acid sequences with an affinity tag; (d) ligating the marked nucleic acid sequences to produce nucleic acid sequences with ligation junctions comprising the affinity tag; (e) reversing the crosslinking of the ligated nucleic acid sequences; (f) shearing the nucleic acid sequences; (g) purifying the sheared nucleic acid sequences with ligation junctions comprising the affinity tag by contacting with a ligand of the affinity tag; (h) A-tailing and amplifying the purified nucleic acid sequences; (i) isolating the amplified nucleic acid sequences that comprise the at least one target nucleic acid sequence or a portion of the at least one target nucleic acid sequence; and (j) sequencing the isolated nucleic acid sequences to identify nucleic acid sequences that interact with the at least one target nucleic sequence; wherein the ligand of the affinity tag in step (g) is attached to a substrate and step (h) is carried out on the substrate.

In an embodiment of the first aspect, the at least one target nucleic acid sequence is selected from the group consisting of promoter, silencer, enhancer, and insulator. In a more particular embodiment, the at least one target nucleic acid sequences is selected from the group consisting of a group of promoters, a group of silencers, a group of enhancers, and a group of insulators. In an even more particular embodiment, the at least one target nucleic acid sequence is a group of promoters.

In an embodiment of the first aspect, the target nucleic acid sequence is a regulatory sequence. In a more particular embodiment, the target nucleic acid sequence is a regulatory sequence selected from the group consisting of promoter, silencer, enhancer, and insulator. As used herein, "regulatory sequence" refers to any nucleic acid sequence that has the capacity to affect the activity status of another genomic sequence.

In a particular embodiment, the at least one nucleic acid sequence is located on the same chromosome as the target nucleic acid sequence; or alternatively, the at least one nucleic acid sequence is located on a different chromosome to the target nucleic acid sequence.

In an embodiment of the first aspect, the nucleic acid composition comprises nucleic acid sequences. In another embodiment, the nucleic acid composition comprises chromosomal DNA. In a more particular embodiment, the chromosomal DNA is derived from, comprises, or consists of, the chromosomal DNA of less than 40 million cells, less than 30 million cells, less than 20 million cells, less than 10 million cells, less than 5 million cells, less than 4 million cells, less than 3 million cells, less than 2 million cells, less than 1 million cells, less than 500.000 cells, less than 400.000 cells, less than 300.000 cells, less than 250.000 cells, less than 200.000 cells, less than 150.000 cells, less than 100.000 cells, less than 90.000 cells, less than 80.000 cells, less than 70.000 cells, less than 60.000 cells, less than 55.000 cells, less than 51.000 cells, or less than 50.000 cells, optionally, wherein the cells are mammalian cells, more particularly human cells.

In an embodiment of the first aspect, the nucleic acid composition is derived from at least one mammalian cell or mammalian cell nucleus; particularly from at least one human cell or nucleus cell nucleus.

It will be appreciated that the method described herein finds utility in a range of organisms, not just humans. For example, the present method may also be used to identify genomic interactions in plants and animals.

In one embodiment of the first aspect, the crosslinking comprises contacting the nucleic acid composition with a crosslinking agent, particularly under suitable conditions. In a particular embodiment, the crosslinking agent comprises an aldehyde; optionally, wherein the aldehyde comprises formaldehyde. In a more particular embodiment, the crosslinking comprises contacting the nucleic acid composition with a formaldehyde under suitable conditions.

References to "crosslinking" or "crosslink" as used herein, refers to any stable chemical association between two compounds, such that they may be further processed as a unit. Such stability may be based upon covalent and/or non- covalent bonding (e.g., ionic). For example, nucleic acids may be crosslinked by chemical agents (i.e., for example, a fixative), heat, pressure, change in pH, or radiation, such that they maintain their spatial relationships during routine laboratory procedures (i.e., for example, extracting, washing, centrifugation etc.). Crosslinking as used herein is equivalent to the terms "fixing" or "fixation", which applies to any method or process that immobilizes any and all cellular processes. A crosslinked/fixed cell, therefore, accurately maintains the spatial relationships between components within the nucleic acid composition at the time of fixation. Many chemicals are capable of providing fixation, including but not limited to, formaldehyde, formalin, or glutaraldehyde. The skilled person knows how to optimize the crosslinking conditions following routine experimentation without the need of any inventive skill.

In an embodiment of the first aspect, the fragmenting in step (b) is selected from the group consisting of restriction endonuclease digestion, sonication, acid incubation, base incubation, microfluidization, and combinations thereof. In a more particular embodiment, the fragmenting in step (b) is digesting with at least one restriction endonuclease.

The term "fragmenting" as used herein, refers to generating nucleic acid sequences shorter than the sequence from which they are derived. Fragments can be of any size, ranging from several megabases and/or kilobases to only a few nucleotides long. Fragments are suitably greater than 5 nucleotide bases in length, for example 10, 15, 20, 25, 30, 40, 50, 100, 250, 500, 750, 1000, 2000, 5000 or 10000 nucleotide bases in length. Fragments may be even longer, for example 1, 5, 10, 20, 25, 50, 75, 100, 200, 300, 400 or 500 nucleotide kilobases in length. The skilled in the art knows several techniques, which belong to the common general knowledge, for fragmenting nucleic acid compositions, and can optimize the fragmenting conditions using routinary tests.

The term "restriction enzyme" as used herein, refers to any protein that cleaves nucleic acid at a specific base pair sequence. Cleavage can result in a blunt or sticky end, depending on the type of restriction enzyme chosen. Examples of restriction enzymes include, but are not limited to, EcoRI, EcoRII, BamHI, Hindlll, Dpnll, Bglll, Ncol, Taql, Notl, Hinfl, Sau3A, Pvull, Smal, Haelll, Hgal, Alul, EcoRV, Kpnl, Pstl, Sad, Sail, Seal, Spel, Sphl, Stul, Xbal. In one embodiment, the restriction enzyme is Hindlll.

The ligation junction with the affinity tag allows ligated fragments to be purified prior to purification step (g), therefore this ensures that non-ligated (i.e., non-interacting) sequences are discarded.

The term "affinity tag" as used herein, refers to any compound or chemical moiety that is capable of being incorporated within a nucleic acid and can provide a basis for selective purification. For example, a junction marker may include, but not be limited to, a labeled nucleotide (e.g., biotin-labeled nucleotide), a modified nucleotide, nick translation, primer linkers, or tagged linkers.

In a particular embodiment, the affinity tag is selected from the group consisting of a labeled nucleotide and polyhistidine. In a more particular embodiment, the affinity tag is a nucleotide labeled with biotin and optionally, the ligand of the affinity tag comprises streptavidin. In another particular embodiment the affinity tag comprises biotin and the ligand of the affinity tag comprises streptavidin. The skilled person knows how to mark the end of nucleotide sequences, for example with a nucleotide labeled with biotin, using molecular biology techniques that belong to the common general knowledge, such as the ones disclosed in the Examples below.

In a particular embodiment of the first aspect, the method is with the proviso that the method does not comprise a step to remove the biotin from the non-ligated ends.

The term "ligating" refers to the generation of any linkage between two nucleic acid sequences, typically through a phosphodiester bond. The linkage is normally facilitated by the presence of a catalytic enzyme (i.e., for example, a ligase such as T4 DNA ligase) in the presence of co-factor reagents and an energy source (i.e., for example, adenosine triphosphate (ATP)). In the method described herein, the fragments of two nucleic acid sequences that have been crosslinked are ligated together in order to produce a single ligated fragment.

In a particular embodiment, the ligating in step (d) comprises contacting the marked nucleic acid sequences with a ligase under suitable conditions.

The term "reversing the crosslinking" as used herein refers to undoing the stable chemical association between the two compounds formed in the crosslinking step. It will be understood that there are several ways known in the art by the skilled person to reverse crosslinks and it will depend upon the way in which the crosslinks are originally formed. For example, crosslinks may be reversed by subjecting the crosslinked nucleic acid composition to high heat, such as above 50°C, 55°C, 60°C, 65°C, 70°C, 75 °C, 80 °C, 85 °C, or greater. Furthermore, the crosslinked nucleic acid composition may need to be subjected to high heat for longer than 1 hour, for example, at least 5 hours, 6 hours, 7 hours, 8 hours, 9 hours, 10 hours or 12 hours or longer.

In one embodiment, reversing the crosslinking in step (e) comprises incubating the crosslinked nucleic acid sequences at 65°C for at least 8 hours in the presence of Proteinase K.

In one embodiment, the shearing in step (f) comprises sonicating. The skilled in the art can perform routinary tests to find the appropriate sonication conditions for obtaining optional results.

In a particular embodiment, the ligand of the affinity tag in step (g) is attached to a substrate. In a more particular embodiment, the ligand of the affinity tag in step (g) is attached to a substrate and step (h) (i.e., A-tailing and amplifying the purified nucleic acid sequences) is carried out on the substrate. In a more particular embodiment, the substrate is a solid substrate. In an even more particular embodiment, the solid substrate is a bead, particularly a magnetic bead.

The substrate can be comprised of any material, for example and without limitation, a solid material, a semisolid material (i.e., a composite of a solid support and a gel) or fluid or liquid material. The substrate can also be comprised of any material that has any dimensions and shape, for example and without limitation, square, trapezoidal, spherical, spheroidal, tubular, pellet-shaped, rod-shaped, or octahedral. The substrate should contain properties that are compatible with the present invention (i.e., exhibit minimal interference with replication, amplification, or detection processes). In certain embodiments, the solid material comprises, for example and without limitation, a glass material (i.e., borosilicate, controlled pore glass, fused silica, or germanium-doped silica), silicon, zirconia, titanium dioxide, a polymeric material (i.e., polystyrene, cross-linked polystyrene, polyacrylate, polymethylacrylate, polydimethylsiloxane, polyethylene, polyfluoroethylene, polyethyleneoxy, polypropylene, polyacrylamide, polyamide such as nylon, dextran, cross-linked dextran, latex, cyclic olefin polymer, cyclic olefin copolymer, as well as other co-polymers and grafts thereof), or a metallic material.

As used herein, the term "A-tailing" refers to the addition of a single deoxyadenosine residue at the end of nucleic acid sequence to form a 3' deoxyadenosine single-base overhang

In one embodiment, the amplifying in step (h) comprises ligating adapters and amplifying by polymerase chain reaction (PCR). The skilled person knows how to design and ligate adapters and how to carry out PCR amplification by routine methods well known in the art. These adapters and methods may be, for instance, those disclosed in the Examples below.

In an embodiment, the method further comprising, after step (e) and before step (f), the step (e') purifying the nucleic acid sequences.

In a more particular embodiment, the method further comprises, after step (e) and before step (f), the step (e') purifying the nucleic acid sequences, with the proviso that a single purification step is carried out between steps (e) and (f). That is, that one single round of purification is carried out between the reversal of the crosslinking and the shearing of the nucleic acids.

There are several techniques in the art, and known to the skilled person, for purifying nucleic acids, for instance by extraction with an organic solvent followed by a precipitation with an alcohol.

Thus, in a particular embodiment, the purifying in step (e') comprises extracting with an organic solvent followed by precipitating with an alcohol. In a more particular embodiment, step (e') comprises extracting with a mixture of phenol, chloroform and isoamyl alcohol followed by precipitating with ethanol. The skilled person knows how to adjust the purification conditions in order to obtain optimal results, particularly when working with low input samples, for instance, by using the conditions disclosed in the Examples below.

In a particular embodiment, the method further comprises, between step (h) and step (i), the step (h') purifying the amplified sequences of length from 100 to 1000 base pairs, from 200 to 900 base pairs, from 300 to 800 base pairs, from 400 to 7000 base pairs, or from 500 to 6000 base pairs. In a more particular embodiment, step (h') comprises purifying the amplified sequences of length from 300 to 800 base pairs. There are several techniques that belong to the common general knowledge that can be used to carry out this size selection, for example those disclosed in the Examples below.

In another embodiment, the method further comprises, between step (f) and step (g), the step (f') end-repairing the sheared nucleic acids.

As used herein, "end-repairing" refers to filling in single stranded overhangs remaining after physically shearing of double stranded nucleic acid fragments, thereby generating blunt ends. Typically, end-repairing comprises contacting the sheared nucleic acid sequences with T4 DNA polymerase, Klenow polymerase, and T4 polynucleotide kinase under suitable conditions.

In an embodiment of the first aspect, the method comprises or consists of the steps (a) crosslinking a nucleic acid composition comprising the at least one target nucleic acid sequence; (b) fragmenting the crosslinked nucleic acid composition, thereby producing fragmented crosslinked nucleic acid sequences; (c) marking the ends of the nucleic acid sequences with an affinity tag;(d) ligating the nucleic acid sequences to produce nucleic acid sequences with ligation junctions comprising the affinity tag; (e) reversing the crosslinking; (e') purifying the nucleic acid sequences; (f) shearing the nucleic acid sequences; (f') end-repairing the sheared nucleic acids; (g) purifying the nucleic acid sequences with ligation junctions comprising the affinity tag by contacting with a ligand of the affinity tag; (h) A-tailing and amplifying the purified nucleic acid sequences; (h') purifying the amplified sequences of length from 100 to 1000 base pairs; (i) isolating the amplified nucleic acid sequences that comprise the at least one target nucleic acid sequence or a portion of the at least one target nucleic acid sequence; and (j) sequencing the isolated nucleic acid sequences to identify nucleic acid sequences that interact with the at least one target nucleic sequence; wherein the ligand of the affinity tag in step (g) is attached to a substrate and step (h) is carried out on the substrate.

In an embodiment of the first aspect, the method comprises, in the following order and consecutively, the steps of: (a) crosslinking a nucleic acid composition comprising the at least one target nucleic acid sequence; (b) fragmenting the crosslinked nucleic acid composition, thereby producing fragmented crosslinked nucleic acid sequences; (c) marking the ends of the nucleic acid sequences with an affinity tag;(d) ligating the nucleic acid sequences to produce nucleic acid sequences with ligation junctions comprising the affinity tag; (e) reversing the crosslinking; (e') purifying the nucleic acid sequences; (f) shearing the nucleic acid sequences; (f') end-repairing the sheared nucleic acids; (g) purifying the nucleic acid sequences with ligation junctions comprising the affinity tag by contacting with a ligand of the affinity tag; (h) A-tailing and amplifying the purified nucleic acid sequences; (h') purifying the amplified sequences of length from 100 to 1000 base pairs; (i) isolating the amplified nucleic acid sequences that comprise the at least one target nucleic acid sequence or a portion of the at least one target nucleic acid sequence; and (j) sequencing the isolated nucleic acid sequences to identify nucleic acid sequences that interact with the at least one target nucleic sequence; wherein the ligand of the affinity tag in step (g) is attached to a substrate and step (h) is carried out on the substrate.

In another embodiment, step (i) comprises contacting the amplified nucleic acid sequences with nucleic acid probes that specifically bind to the at least one target nucleic acid sequence, and purifying the nucleic acid probes bound to the nucleic acid sequences that comprise the at least one target nucleic acid sequence or a portion of the at least one target nucleic acid sequence. In a more particular embodiment, the nucleic acid probes are selected from DNA, cDNA, and RNA probes. In a more particular embodiment, the nucleic acid probes are RNA probes. In another particular embodiment, the nucleic acid probes are labelled. In an even more particular embodiment, the nucleic acid probes are, biotinylated RNA probes.

References herein to a "nucleic acid probe" refers to a molecule formed of nucleic acids which specifically bind to the target nucleic acid sequence. For example, the nucleic acid probe may contain the complementary sequence to the target nucleic acid segment which will then form interactions with the nucleotide bases of the target nucleic acid segment i.e., to form base pairs (bp)). It will be understood that the nucleic acid probe, for example biotinylated RNA, does not need to contain the entire complementary sequence of the target nucleic acid segment in order to form complementary interactions and isolate it from the nucleic acid composition. The isolating nucleic acid molecule may be at least 10 nucleotide bases long, for example, at least 20, 30, 40, 50, 60, 70, 80, 90, 100, 130, 150, 170, 200, 300, 400, 500, 750, 1000, 2000, 3000, 4000 or 5000 nucleotide bases long.

In one embodiment, the sequencing in step (j) comprises ligating paired end adapter sequences to the ends of the isolated target ligated sequences and then sequencing. The term "paired end adapters" as used herein, refers to any primer pair set that allows automated high throughput sequencing to read from both ends simultaneously. For example, such high throughput sequencing devices that are compatible with these adaptors include, but are not limited to Solexa (Illumina), the 454 System, and/or the ABI SOLiD.

In one embodiment of the first aspect, steps (a) to (e) are carried out in a single reaction tube. In one embodiment of the first aspect, steps (g) to (h), and optionally (h'), are carried out in a single reaction tube. In another embodiment, steps (a) to (e) are carried out in a first reaction tube, and steps (g) to (h), and optionally (h'), are carried out in a second reaction tube.

As above explained, in a second aspect the invention provides a method of identifying one or more interacting nucleic acid sequences that are indicative of a particular disease state.

References to "frequency of interaction" or "interaction frequency" as used herein, refers to the number of times a specific interaction occurs within a nucleic acid composition (i.e., sample). In some instances, a lower frequency of interaction in the nucleic acid composition, compared to a normal control nucleic acid composition from a healthy subject, is indicative of a particular disease state (i.e., because the nucleic acid segments are interacting less frequently). Alternatively, a higher frequency of interaction in the nucleic acid composition, compared to a normal control nucleic acid composition from a healthy subject, is indicative of a particular disease state (i.e., because the nucleic acid segments are interacting more frequently). In some instances, the difference will be represented by at least a 0.5-fold difference, such as a 1-fold, 1.5-fold, 2-fold, 2.5-fold, 3-fold, 4-fold, 5-fold, 7-fold or 10-fold difference.

The step (ii) quantifying can be performed by any method suitable to calculate the frequency of interaction in a nucleic acid composition from a patient or a purification or extract of a nucleic acid composition sample or a dilution thereof. For example, high throughput sequencing results can also enable examination of the frequency of a particular interaction. In methods of the invention, quantifying may be performed by measuring the concentration of the target nucleic acid segment or ligation products in the sample or samples. The nucleic acid composition may be obtained from cells in biological samples that may include cerebrospinal fluid (CSF), whole blood, blood serum, plasma, or an extract or purification therefrom, or dilution thereof. In one embodiment, the biological sample may be cerebrospinal fluid (CSF), whole blood, blood serum or plasma. Biological samples also include tissue homogenates, tissue sections and biopsy specimens from a live subject, or taken post-mortem. The samples can be prepared, for example where appropriate diluted or concentrated, and stored in the usual manner.

In one embodiment, the disease state is selected from the group consisting of cancer, autoimmune disease, a developmental disorder, a genetic disorder, diabetes, cardiovascular disease, kidney disease, lung disease, liver disease, neurological disease, viral infection, and bacterial infection. In a further embodiment, the disease state is cancer or autoimmune disease. In a yet further embodiment, the disease state is cancer, for example breast, bowel, bladder, bone, brain, cervical, colon, endometrial, esophageal, kidney, liver, lung, ovarian, pancreatic, prostate, skin, stomach, testicular, thyroid or uterine cancer, leukemia, lymphoma, myeloma or melanoma. References herein to an "autoimmune disease" include conditions which arise from an immune response targeted against a person's own body, for example Acute disseminated encephalomyelitis (ADEM), Ankylosing Spondylitis, Behget's disease, Celiac disease, Crohn's disease, Diabetes mellitus type 1, Graves' disease, Guillain-Barre syndrome (GBS), Psoriasis, Rheumatoid arthritis, Rheumatic fever, Sjogren's syndrome, Ulcerative colitis and Vasculitis.

References herein to a "developmental disorder" include conditions, usually originating from childhood, such as learning disabilities, communication disorders, Autism, Attention-deficit hyperactivity disorder (ADHD) and Developmental coordination disorder.
References herein to a "genetic disorder" include conditions which result from one or more abnormalities in the genome, such as Angelman syndrome, Canavan disease, Charcot-Marie-Tooth disease, Color blindness, Cri du chat syndrome, Cystic fibrosis, Down syndrome, Duchenne muscular dystrophy, Haemochromatosis, Haemophilia, Klinefelter syndrome, Neurofibromatosis, Phenylketonuria, Polycystic kidney disease, Prader-Willi syndrome, Sickle-cell disease, Tay-Sachs disease and Turner syndrome.

As commented above, in a third aspect the invention provides a kit of parts. It will be understood that examples of the types of buffers and reagents to be included in the kit can be seen in the Examples described herein. For example, an oligonucleotide probe and/or pair of amplification primers for use in the methods described herein may be provided in isolated form and may be part of a kit, e.g., in a suitable container such as a vial in which the contents are protected from the external environment. The kit may include instructions for use according to the protocol of the method described herein. A kit wherein the nucleic acid is intended for use in PCR may include one or more other reagents required for the reaction, such as polymerase, nucleotides, buffer solution etc.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

### Materials and Methods

### Cell isolation

Naive B cells, naive CD4+ cells, naive CD8+ cells, megakaryocytes, erythroblasts and monocytes were isolated as described in Javierre, B. M. et al. Lineage-Specific Genome Architecture Links Enhancers and Non-coding Disease Variants to Target Gene Promoters. Cell 167, 1369-1384.e19 (2016) from human donors.

Hematopoietic stem and progenitor cells (HSC; CD34+, CD38-) common myeloid progenitors (CMP; CD34+, CD38+, CD33+) and common lymphoid B cell progenitors (CLP; CD34+, CD38+, CD19+) were purified by CD34 positive selection (Miltenyi cat. #130-046-703) and fluorescence-activated cell sorting (FACS) from 15- to 22-week-old human fetal liver (FL) and fetal bone marrow (FBM) as previously described in Anindita, R. et al. Perturbation of fetal liver hematopoietic stem and progenitor cell development by trisomy 21. Proc. Natl. Acad. Sci. 109, 17579-17584 (2012). Briefly, after CD34+ selection, positive cells were stained for flow cytometry with the following fluorophore-conjugated monoclonal antibodies, all from BD Biosciences: CD34 PECy7 (cat. #348811), CD38 FITC (cat. #555459), CD19 BV421 (cat. #562440), CD33 APC (cat. #551378) and CD10 PE (cat. #555375). FACS was performed using a BD FACSAria Fusion. B-ALL samples were isolated from bone marrow of pediatric patients after CD19+by FACS sorter.

### Fixation and cell quantification

Cells were quantified, resuspended in 1ml of RPMI 1640 culture medium containing 10% FBS and 2% of methanol-free formaldehyde (Thermo Fisher cat. #28908) and incubated in a rocker for 10 minutes at room temperature. Formaldehyde was quenched with glycine to a final concentration of 0.125M. Then, cells were washed with cold 1X PBS. Specific cell numbers were sorted into low-retention 1.5ml tubes (BD FACSJazz sorter, 1.0 Drop Pure sorting mode), pelleted, flash-frozen in dry ice and stored at -80 °C.

### Low input capture Hi-C (liCHi-C) method

During the whole protocol, low-retention tips and tubes were used to minimize cell loss. Pelleted cells were softly resuspended in 500µl ice cold lysis buffer (10mM Tris-HCI pH 8.0, 10mM NaCl, 0.2% IGEPAL CA-630, 1x cOmplete EDTA-free protease inhibitor cocktail (Merck cat. #11873580001)) and incubated 30 minutes on ice to extract the nuclei. Soft inversions of the tube were performed during the incubation every 5 minutes. Nuclei were centrifuged (1000g and 4°C for 10 min) and 450µl of supernatant was discarded (50µl of supernatant was left in the tube to avoid cell losses). The cell pellet was resuspended in 500µl ice cold 1.25x NEB2 buffer (New England Biolabs cat. #B7002S). After centrifugation (1000g and 4°C for 10 min), 500µl of supernatant was removed.

Afterwards, 129ul of 1.25x NEB2 buffer were added to the low-retention tube to obtain a final volume of 179ul. Then, 5.5µl of 10% SDS (AppliChem cat. #A0676,0250) were laid on the wall of the tube and mixed by inversion. After incubation at 37°C and 950 rpm for 30 minutes, 37.5µl 10% Triton X-100 (AppliChem cat. #A4975,0100) were laid on the wall of the tube, mixed by inversion and incubated at 37°C and 950 rpm for 30 minutes. Chromatin within the nuclei was overnight digested at 37°C and 950rpm after adding 7.5µl of HindIII restriction enzyme at 100U/µl (New England Biolabs cat. #R0104T). The following day, an extra digestion during one more hour was performed after adding 2.5µl of the HindIII enzyme.

After digestion, cohesive restriction fragment ends were filled in during 75 minutes at 37°C. To do so, 30ul of master mix composed by 5µl of 5U/µl Klenow polymerase (New England Biolabs cat. #M0210L), 0.75µl of each 10mM dCTP, dGTP and dTTP, and 18.75µl of 0.4mM biotin-14-dATP (Invitrogen #19524-016) were added.

In-nucleus ligation of DNA fragments was carried out during 4 hours at 16°C after adding 12.5µl of 1U/ul T4 DNA ligase (Thermo Fisher cat. #15224025), 50µl of 10x ligation buffer (NEB #B0202S), 5µl of 10mg/ml BSA (NEB # B9001S) and 170.5ml of water. Afterwards, DNA ligation products were decrosslinked by adding 30ul of Proteinase K 10mg/ml (Merck cat. #3115879001) and incubating overnight at 65°C. The following day, an extra decrosslink during two more hours was performed after adding 15µl of the Proteinase K enzyme.

To purify the decrosslinked DNA ligation products, a single phenol-chloroform-isoamyl alcohol (25:24:1 v/v) purification was carried out followed by ethanol precipitation for 1 hour at -80°C in presence of 30ug Glycoblue (Thermo Fisher cat. #AM9515) as a coprecipitant. DNA ligation products were resuspended in 130ul of nuclease free water and concentration was assessed by fluorimetric quantification using the Qubit dsDNA HS Assay Kit (Thermo Fisher cat. #Q32851).

Biotin removal of the non-ligated ends was skipped. DNA ligation products were sonicated using Covaris M220 focused-ultrasonicator (20% duty factor, 50 peak incident power, 200 cycles per burst, 65 seconds) in 130ul tubes (Covaris cat. #520077). After shearing, DNA ends were repaired by adding 6.5ul of T4 DNA polymerase 3U/ul (New England Biolabs cat. #M0203L), 6.5ul of T4 polynucleotide kinase 10U/ul (New England Biolabs cat. #M0201L) 1.3ul of Klenow polymerase 5U/ul (New England Biolabs cat. #M0210L), 18ul of dNTP mix 2.5mM each and 18ul of 10x ligation buffer (New England Biolabs cat. #B0202S) and incubating for 30 minutes at 20°C.

Biotinilated informative DNA ligation products were pulled down using Dynabeads MyOne streptavidin C1 paramagnetic beads (Thermo Fisher cat. #65001). After thorough washing of the ligation products-beads complex and having the sample in 35.7ul of volume, blunt DNA fragments on the beads were adenine-tailed by adding 7ul of Klenow 3'→5' exo- polymerase 5U/ul (New England Biolabs cat. #M0212L), 2.3ul of dATP 10mM and 5ul NEB2 of 10x NEB 2 Buffer and incubating the mixture 30 minutes at 37°C and a further 10 minutes at 65°C to inactivate the enzyme.

After thorough washing of the ligation products-beads complex and having the sample in 50ul of 1x ligation buffer, PE Illumina adapters (PE1 Adapter: 5' GATCGGAAGAGCGGTTCAGCAGGAATGCCGAG 3' (SEQ ID NO: 1); PE2 Adapter; 5' ACACTCTTTCCCTACACGACGCTCTTCCGATCT 3' (SEQ ID NO: 2)) were ligated to the adenine-tailed DNA fragments by adding 1ul of T4 DNA ligase 2000U/ul (New England Biolabs cat. #M0202T) and 4ul of preannealed adaptor mix 15uM and incubating the mixture 2h at room temperature.

The bead-bound ligation products were amplified 8-13 cycles by PCR (PE PCR Primer 1: 5' AATGATACGGCGACCACCGAGATCTACACTCTTTCCCTACACGACGCTCTTCCGATCT 3' (SEQ ID NO: 3); PE PCR Primer 2: 5' CAAGCAGAAGACGGCATACGAGATCGGTCTCGGCATTCCTGCTGAACCGCTCTTCCGATCT 3' (SEQ ID NO: 4)) using Phusion high-fidelity PCR master mix with HF buffer (New England Biolabs cat. #M0531L)

After recovering the amplified library from the supernatant, size distribution was tailored to 300-800bp by double-sided size selection and purified using CleanNGS SPRI beads (0.4-1 volumes; CleanNA cat. #CNGS-0050) according to manufacturer's instructions. DNA concentration was quantified on an Agilent Tapestation platform using high sensitivity D1000 ScreenTape system and samples were stored at -20°C.

Enrichment of promoter-containing ligation products was performed using SureSelectXT Target Enrichment System for the Illumina Platform (Agilent Technologies) as instructed by the manufacturer, and the library was amplified 4 cycles by PCR using Phusion high-fidelity PCR master mix with HF buffer (New England Biolabs cat. #M0531L). Finally, the end product was purified using CleanNGS SPRI beads (0.9 volumes; CleanNA cat. #CNGS-0050) and paired-end sequenced.

### Sequencing

B-ALL liCHi-C libraries were sequenced by Macrogen Inc using HiseqX 150+150PE platform. The rest of liCHi-C libraries were sequenced by BGI Genomics using DNBseq 100+100PE platform.

### B-ALL cytogenetic and FISH analysis

Cytogenetic analyses of B-ALL samples were carried out on G-banded chromosome obtained from 24-hour unstimulated culture. FISH analyses were performed on fixed cell suspensions of the bone marrow (B-ALL 1) and on a bone marrow smear (B-ALL 2) using the LSI MYC probe (Metasystems, XL MYC BA) and the LSI IGH probe (Metasystems, XL IGH BA), respectively. Around 400 and 200 interphase nuclei interphase nuclei for B-ALL 1 and B-ALL 2 respectively were scored.

### liCHi-C processing

Paired-end reads were processed using HiCUP (0.8.2) (Wingett, S. W. et al. "HiCUP: pipeline for mapping and processing Hi-C data". F1000Research 4, (2015)). First, the genome was computationally digested using the target sequence of the Hindlll enzyme. Then, the different steps of the HiCUP pipeline were applied to map the reads to the human genome (GRCh38.p13), filter out all the experimental artifacts and remove the duplicated reads and retain only the valid unique paired reads. To assess the capture efficiency, those paired reads which any end overlaps with a captured restriction fragment were filtered out, retaining only the unique captured valid reads for further analysis.

### liCHi-C interaction calling

Interaction confidence scores were computed using the CHiCAGO R package as described in Cairns, J. et al. "CHiCAGO: robust detection of DNA looping interactions in Capture Hi-C data". Genome Biol. 17, 127 (2016). In summary, this pipeline implements a statistical model with two components (biological and technical background), together with normalization and multiple testing methods for capture Hi-C data. CHiCAGO analysis was performed in merged samples to increase the sensitivity, after assessing for reproducibility between biological replicates using: i) the stratum adjusted correlation coefficient according to Yang, T. et al. "HiCRep: assessing the reproducibility of Hi-C data using a stratum-adjusted correlation coefficient". Genome Res. 27, 1939-1949 (2017), averaged over chromosomes, ii) principal component analysis, and iii) hierarchical clustering. The reproducibility-score was also used to compare PCHi-C and liCHi-C libraries of the same cell types. Significant interactions with a CHiCAGO score ≥ 5 were considered as high confidence interactions.

### Clustering of promoter interactions

Interactions were clustered using the Autoclass algorithm and for each cluster a specificity score was computed as described in Javierre, B. M. et al. *supra* using the asinh-transformed CHiCAGO scores. Clustering of cell types was performed using a hierarchical method with average linkage based on Euclidean distances, and principal component analysis was performed using the prcomp function in R. For interaction data handling we used an in-house R package which allows us to compute distance distributions, filter interactions by the presence of histones marks, or generate virtual 4C of specific genes.

### ChIP-seg processing

Paired-end reads were processed following ENCODE standards. Reads were trimmed using Trim Galore (0.6.5) to remove sequencing adapters, and then mapped using bowtie2 (2.3.2) to the reference genomeat PRS (GRCh38.p13) with -very-sensitive parameter. Low quality reads, reads overlapping the ENCODE blacklist and duplicated reads were filtered out. Peak calling was performed using macs2 (2.2.7.1) in broad and narrow mode depending on the histone mark using an input sample as control, with default parameters. Bigwig files were generated using the function bamCoverage from deepTools (3.2.1) and scaled based on the background normalization of the samples before merging the biological replicates together for visualization purposes.

### Histones mark enrichment at PIRs

Enrichment of the different histones mark ChIP-seq in both promoters and npPIRs was performed using a permutation test implemented in GenoMatriXeR (https://github.com/RMalinverni/GenoMatriXeR), with 5000 randomizations using the randomizeRegions option. Promoter interactions were classified based on the RPKM expression quartiles of their genes, and the promoters and npPIRs analyzed separately, generating four groups of either promoters or npPIRs from lower expression to higher expression.

### Chromatin assortativity

Chromatin assortativity was computed for the promoter-pPIR and promoter-npPIR subnetworks separately using ChAseR R package as described in Madrid-Mencia, M., et al. "Using GARDEN-NET and ChAseR to explore human haematopoietic 3D chromatin interaction networks". Nucleic Acids Res. 48, 4066-4080 (2020). The results were validated against a set of 1000 randomizations preserving the genomic distances between nodes and the chromosomes distribution of interactions. The abundance of each histone mark on the nodes of the networks was also computed.

### Gene ontology and pathways enrichment analysis

Gene ontology (GO) enrichment analysis was performed using clusterProfiler R package for the 3 ontologies: molecular functions, biological processes and cellular components. The pathway analysis was done using ReactomePA. It was used the prioritized protein-coding genes by COGS (gene score >= 0.5) to compute enrichment in genes in Reactome pathways, adjusting p values by false discovery rate. In both analyses it was used as universe all the genes in the capture design.

### Data visualization

To visualize the contact matrices, the bam files, containing unique captured valid reads, were transformed into pair files using bam2pairs. Then they were converted into matrices in cool format with a resolution of 1Mb, 250kb and 100kb using cooler (0.8.11). The visualization of the matrices was done using HiCExplorer (v3.6). For the visualization of significant interactions generated by liCHi-C we used the WashU Epigenome Browser and karyoploteR R package.

### GWAS summary statistics and imputation

GWAS summary data was obtained from the NHGRI-EBI GWAS Catalog and from the UK Biobank - Neale Lab (UK Biobank, *n.d.;* http://www.nealelab.is/uk-biobank). Those datasets that were not in GRCh38 coordinates were converted to it using *liftOver.* To avoid spuriously strong association statistics, it was filtered out SNPs with p value < 5·10⁻⁸ for which there were no SNPs in LD (r₂ > 0.6 using 1000 genomes EUR cohort as a reference genotype set or within 50kb with p value < 10⁵. To increase the power of the GWAS we applied the Poor Man's Imputation to the summary statistics described above using as reference genotype set the 1000 Genomes EUR cohort. It was used the GRCh38 HapMap Phase II genetic map lifted from GRCh37 coordinates to define regions with 1cM recombination frequency to be used for the imputation. The MHC region (GRCh38:6:25-35Mb) was excluded from the analysis. Manhattan plots were visualized using the qqman package (v0.1.8).

### GWAS enrichment at PIRs

Enrichment of SNPs in PIRs was performed using Blockshifter which considers the correlation between GWAS and PIRs. Blockshifter computes a z-score using a competitive test for each trait and cell type set.

### liCHi-C GWAS prioritizing genes

Prioritization of relevant genes for each GWAS trait and cell type was performed using the COGS algorithm. Briefly, this method considers linkage disequilibrium to estimate the posterior probability of each SNP being casual for each trait. Then these SNPs were used to compute a gene-score for all the gene involved in liCHi-C significant interactions in at least one cell type. This gene-score is composed by 3 components: coding SNPs annotated by VEP (104); SNPs located in promoter regions; and SNPs overlapping Other-Ends.

### SVs analysis

Translocations of B-ALL samples were identified using PLlER_briefly it compares the genome-wide interactions of a region against a set of random permutations computing a z-score. To detect CNVs, it was applied BIC-seq2, an algorithm that uses Bayesian information criterion-based segmentation on normalized data using naive B samples as control genome. Visual inspection of both translocations and CNVs was performed by computing the log2ratio between the contact matrices of B-ALL and CLP samples.

### Results

### Development and optimization of the method of the invention (liCHi-C) for low-input samples

In order to enable the detection of the promoter interactome using low-input material, previous Hi-C methods were extensively modified to develop the method of the invention (liCHi-C), which maximizes library complexity by eliminating and modifying the sequence of some steps (Fig. 1A). Additionally, it reduces by half the time spent on the library preparation.

To systematically evaluate liCHi-C, libraries were generated from decreasing numbers of human naive B cells at controlled ratios, and compared these with the most comprehensive promoter capture Hi-C (PCHi-C) data available to date from the exact same cell type that used ~40 M cells as starting material. Each sample was deep-sequenced and paired-end reads were mapped and filtered using HiCUP pipeline as above described. Visual inspection of normalized liCHi-C and PCHi-C contact maps showed a high degree of similarity in topological properties (Fig. 2b). For all experimental conditions (i.e., number of starting cells), the percentage of valid reads (Mean = 58.41 %; SD = 5.76%) and the capture efficiency (Mean = 61.29%; SD = 9.15%) were surprisingly similar between both methods, being 23.58% (SD = 1.59%) of those contacts in *trans* (Fig. 2c-e). Interaction matrices were also highly reproducible for both methods and different amounts of starting material (stratum-adjusted correlation coefficient (SCC) > 0.90) (Fig. 1b and Fig. 2f).

These data suggest that liCHi-C reliably generate high-quality promoter interactomes, including 31,253 annotated promoters, and can routinely be performed successfully from as low input as 50,000 (50k) cells. Remarkably, liCHi-C was able to achieve a >10-fold enrichment of read pairs involving promoters when compared with Hi-C using 800 times less of the starting cells.

### Comparison of liCHi-C with PCHi-C for profiling promoter interactomes

To formally compare the performance of liCHi-C to detect promoter interactions, CHiCAGO pipeline was used as described above to call for significant interactions (CHiCAGO score > 5). Distance distribution and nature of interacting fragments were similar across cell number conditions and methods (Fig. 3a-c). Specifically, an average median linear distance between promoters and their interacting regions of 265kb (SD = 30kb), and 89.04% (SD = 3.72%) of these were promoter-to-non-promoter interactions were found (Fig. 3b-c). Principal component analysis (PCA) of CHiCAGO interaction scores across all biological replicates demonstrated that patterns of promoter interactions were highly consistent across biological replicates and group samples according to the number of input cells (Fig. 1c). To further explore the limits of liCHi-C library complexity hierarchical clustering based on their CHiCAGO interaction scores was performed. Promoter interactomes generated from >100k cells reproduce the ones generated with PCHi-C on 40,000,000 (40M) cells (Fig. 3d). Although promoter interactomes with less than 100k cells showed a different clustering profile, potentially reflecting the reduction of significant interactions due the limited library complexity, these retain cell-type specific and invariant topological features (Fig. 1d and Fig 3e-g).

Collectively, these results demonstrate the high reproducibility of liCHi-C to profile promoter interactomes at a similar resolution as PCHi-C, and underscore the suitability of the approach to investigate these interactions in cell populations present at relatively low abundance within a sample.

### liCHi-C efficiently captures promoter interactomes across different hematopoietic lineages

To assess the capacity of liCHi-C to provide fundamental insight on *in vivo* cell differentiation, high-quality liCHi-C experiments were performed in 9 distinct cellular populations from the human hematopoietic hierarchy (two biological replicates per cell type), including hematopoietic stem and progenitor cells (HSC), common myeloid progenitors (CMP), common lymphoid B cell progenitors (CLP) and 6 differentiated cell types (Fig. 4a-c). As a more comprehensive validation of liCHi-C method, it was first focused on the differentiated cell types for which high quality PCHi-C data was available. Benchmarking of liCHi-C data against PCHi-C data demonstrated high reproducibility between both methods for all cell types profiled (SCC > 0.93) (Fig. 5a and Fig. 4c). Moreover, promoter interactomes clearly separated cell types independently of the method used or the starting number of cells (Fig. 4f).

liCHi-C data was then analyzed in detail. Applying CHiCAGO a median of 134,965 high-confidence promoter interactions (CHiCAGO score > 5) per cell type were identified (Fig. 4d-e. A PCA of interaction scores demonstrated that promoter interactomes were highly reproducible and dynamic (Fig. 5b). While the first principal component separated cells according to their myeloid or lymphoid linage, the second principal component recapitulated the differentiation potential, enabling altogether to identify both myeloid and lymphoid differentiation trajectories. To decipher these specificities in greater depth, Autoclass Bayesian clustering was applied and computed the specificity score of each cluster in each cell type (Fig. 5c-d). Among the 33 clusters, a stem and progenitor-specific cluster (C3), lymphoid-specific clusters (e.g., C10, C13), myeloid-specific clusters (e.g., C25, C27) and cell-type specific clusters (e.g., C4, C7) that contained genes known to be involved in cellular functions important for the given cell types were observed (Fig. 5d-g). These observations were clearly illustrated by the promoter interactomes of *MYB,* which encodes for transcription regulator that plays an essential role in the regulation of lymphoid priming and early B cell development (Fig. 5e), *ITGA2B,* which encodes for the megakaryocyte-specific surface marker CD41 (Fig. 5f), and *SARS2,* a housekeeping gene that encodes for the mitochondrial seryl-tRNA synthetase (Fig. 5g). Collectively, these data demonstrate that promoter interactomes are specific to the differentiation trajectories of cell types, and further suggest that the highly dynamic promoter-centric genome architecture recapitulates the developmental history of hematopoietic cell lineages.

### Promoter interactomes reshape transcriptional trajectories during in vivo cell commitment

To validate the ability of liCHi-C to uncover mechanistic insights on transcription regulation, it was computationally integrated promoter interactome data with RNA-seq and ChIP-seq data from matched cell types. It was distinguished between 2 types of promoter-interacting regions (PIRs): non-promoter PIRs (npPIRs), in which the promoter interacting region does not contain any captured gene promoter, and promoter PIRs (pPIRs), in which the promoter interacting region contains at least a gene promoter. It was found high enrichment of histone modifications indicative of active enhancers (e.g., H3K27ac, H3K4me1) and non-coding transcription of regulatory regions (e.g., H3K4me3) at distal npPIRs. These enrichments were positively associated with expression level of linked genes in a cell-type specific manner. Conversely, npPIRs of low-expressed genes tend to be more enriched in repressive histone marks, such as H3K27me3 and H3K9me3, than higher expressed ones. These enrichment profiles were highly consistent with the ones obtained with PCHi-C data despite of the significant difference in the starting cell number.

Collectively, these results, exemplified by the transcriptional regulation of the T cell-specific gene GATA3, the B cell-specific gene *PAX5,* and the HSC-specific gene CD34, demonstrate the capacity of liCHi-C to identify distal regulatory elements for each gene in rare cell types hitherto undeterminable, and suggest that promoter-associated regions are enriched in distal regulatory elements that mirror the cell-type specificity of the interacting gene's expression.

It was then applied chromatin assortativity analysis, which recognizes the preference of a network's nodes to attach to others that have similar features, to test the potential of liCHi-C to discover proteins or chromatin marks mediating genomic contacts within the nucleus. It was observed that genomic regions enriched in H3K9me3 histone modification, which have been associated with constitutive heterochromatin and lamina-associated domains, are highly interconnected and may form topological hubs that collaborate with epigenetics to promote gene silencing.

Collectively, these results illustrate the power of liCHi-C to expose new aspects on the diversity of factors and mechanisms regulating genome architecture in physiological and pathogenic settings.

### liCHi-C enables the discovery of disease-relevant cell types and disease-associated genes and pathways

Genetic variation, which frequent affects the non-coding genome, occurs at various levels ranging from single-nucleotide variants, such as single-nucleotide polymorphisms (SNPs), to larger structural variants (SVs). To test liCHi-C's ability to uncover novel associations between non-coding SNPs and disease etiology, summary statistics from thirty-nine genome-wide association studies (GWAS) were integrated, including seven autoimmune diseases, eight myeloid cell traits, four lymphoid cell traits, nine blood malignancies and eleven traits non-related with the hematopoietic hierarchy. Using Blockshifter analysis it was shown that cell-type specific PIRs called by liCHi-C were enriched for genetic variants from association studies of traits or diseases with similar cell specificity. For instance, variants associated with final maturation of myeloid cells tend to be more enriched at PIRs in mature myeloid cells. Interestingly, similar enrichment profiles were obtained by liCHi-C and PCHi-C despite the dramatic reduction in starting material used in liCHi-C. These data demonstrate that liCHi-C can trace the ontogeny of activity of the non-coding genome in association with pathogenic traits, and enables the identification of cell types presumably implicated in disease etiology.

Bayesian prioritization strategy COGS was then used to rank putative disease-associated genes based on GWAS and liCHi-C data. Excluding SNPs at promoter or coding regions, 24,504 distal non-coding SNPs were assigned to potential target genes, which were located at a median genomic distance of 187 kb. Remarkably, only 19.58% of these were linked to the nearest gene and 38.16% potentially controlled more than one gene.

These results highlight the importance of being able to generate data on long-range interactions between promoters and regulatory elements to avoid misleading associations based on proximity in the context of gene regulation and disease.

Specifically, using this computational framework on liCHi-C data 6,230 candidate genes were prioritized (with a median of 134 genes per trait/disease at gene-level score > 0.5) and 56 candidate gene pathways according the Reactome Pathway Database. These genes were highly similar to those prioritized by PCHi-C using 40M cells.

These results demonstrate the power of liCHi-C to identify potential disease-causative genes and pathways.

### liCHi-C can be used to simultaneously diagnose and discover translocations, copy number variations (CNVs) and topological alterations in tumoral samples

After demonstrating the capacity of liCHi-C to prioritize non-coding SNPs with potential functional relevance in clinical settings, it was investigated SVs affecting larger genomic regions, including translocations and CNVs. Most of the ligation events detected by proximity-ligation methods, such as liCHi-C, occur between contiguous sequences in the linear genome and the frequency of these events decreases logarithmically with genomic distance. However, a translocation alters the linear genome and artificially increases the number of ligation events between the juxtaposed regions. High quality liCHi-C libraries were generated using primary blasts from two pediatric B cell acute lymphoblastic leukemia (B-ALL) samples previously analyzed by routine clinical assays. According to FISH and karyotyping analysis, B-ALL sample 1 carried a balanced translocation between chromosomes 8 and 14, which appeared as 'butterfly' blocks of interactions between the translocated chromosomes on the liCHi-C interaction matrix. A closer examination of the interaction directionalities identified the restriction fragments affected by the breakpoints and allowed the reconstruction of the resulting chromosome where the promoter of *MYC* became rearranged next to the "constant" gene cluster of the IgH locus. Through this focused analysis of promoter interactions, these data suggested that *MYC* expression may be simultaneously controlled by the *"MYC* blood enhancer cluster" (BENC), located 2Mb downstream, and the IgH-specific enhancer 3'-regulatory region (3'-RR), rearranged 300kb upstream. B-ALL sample 2 carried an unbalanced translocation between the same chromosomes that generated single blocks of contacts on the normalized liCHi-C interaction matrix. Consequently, the *CEBPD* gene becomes juxtaposed to the IgH locus, which potentially alters its regulatory landscape.

Collectively, these results demonstrate liCHi-C's capacity to generate high-quality chromatin conformation maps and regulatory landscapes directly from primary patient tissue samples to detect any type of translocation and surmise the pathogenic effects at a genome-wide scale.

In addition to chromosome rearrangements, it was then tested whether liCHi-C could be used to detect CNVs. B-ALL sample 2 disclosed above that, according to the karyotyping analysis, carried a trisomy of chromosome 21 and a partial trisomy affecting the translocated region of the q arm of chromosome 8 was analyzed. Both trisomies were identified with liCHi-C, demonstrating that liCHi-C can be used for genome-wide detection of CNVs and scan breakpoints from primary patient tissues without the need for a reference.

Finally, the ability of liCHi-C to identify disease-specific regulatory 3D chromatin landscapes that may be implicated in disease etiology was tested. To do so, Autoclass Bayesian clustering of liCHi-C significant interactions (CHiCAGO score > 5) called either on B-ALL samples or on the postulated healthy cells of origin of these hematological malignancies (i.e., HSC, CLP and naive B cells) was applied. Specificity score analysis of each cluster in each cell type identified promoter interactions specific acquired (C5-9) or lost (C10-19) in one or both B-ALL samples, which included key transcription factors involved in B cell differentiation and function (*e.g., PAX5, ARID5B*) and well-known tumor suppressor genes (*e.g., BTG1, IKZF1*) and protooncogenes (*e.g., MYC*)*.* For instance, several *HOXA* genes have been associated with normal hematopoiesis and blood malignancies. According to liCHi-C data, these genes lost connectivity with their enhancers, which could link their transcriptional deregulation with malignant transformation.

Collectively these results support the broad applicability of liCHi-C to uncover factors and mechanisms involved in disease etiology through simultaneously identifying disease-specific promoter-centered genome topologies and detecting translocations, CNVs, breakpoints and their effects on transcriptional deregulation.

### Citation List

Javierre, B. M. et al. "Lineage-Specific Genome Architecture Links Enhancers and Non-coding Disease variants to Target Gene Promoters". Cell 167, 1369-1384.e19 (2016)
Anindita, R. et al. "Perturbation of fetal liver hematopoietic stem and progenitor cell development by trisomy 21". Proc. Natl. Acad. Sci. 109, 17579-17584 (2012)
Wingett, S. W. et al. "HiCUP: pipeline for mapping and processing Hi-C data". F1000Research 4, (2015)
Cairns, J. et al. "CHiCAGO: robust detection of DNA looping interactions in Capture Hi-C data". Genome Biol. 17, 127 (2016)
Yang, T. et al. "HiCRep: assessing the reproducibility of Hi-C data using a stratum-adjusted correlation coefficient". Genome Res. 27, 1939-1949 (2017)
Torbey, P. et al. "Cooperation, cis-interactions, versatility and evolutionary plasticity of multiple cis-acting elements underlie krox20 hindbrain regulation". PLOS Genet. 14, e1007581 (2018)
Madrid-Mencia, M., et al. "Using GARDEN-NET and ChAseR to explore human haematopoietic 3D chromatin interaction networks". Nucleic Acids Res. 48, 4066-4080 (2020)

## Claims

1. A method for identifying at least one nucleic acid sequence that interacts with at least one target nucleic acid sequence, the method comprising the steps of:
(a) crosslinking a nucleic acid composition comprising the at least one target nucleic acid sequence;
(b) fragmenting the crosslinked nucleic acid composition, thereby producing fragmented crosslinked nucleic acid sequences;
(c) marking the ends of the nucleic acid sequences with an affinity tag;
(d) ligating the nucleic acid sequences to produce nucleic acid sequences with ligation junctions comprising the affinity tag;
(e) reversing the crosslinking;
(f) shearing the nucleic acid sequences;
(g) purifying the nucleic acid sequences with ligation junctions comprising the affinity tag by contacting with a ligand of the affinity tag;
(h) A-tailing and amplifying the purified nucleic acid sequences;
(i) isolating the amplified nucleic acid sequences that comprise the at least one target nucleic acid sequence or a portion of the at least one target nucleic acid sequence; and
(j) sequencing the isolated nucleic acid sequences to identify the at least one nucleic acid sequence that interact with the at least one target nucleic sequence;
with the proviso that the method does not comprise a step to remove the affinity tag from non-ligated ends.

2. The method according to claim 1, wherein the at least one target nucleic acid sequence is selected from the group consisting of promoter, silencer, enhancer, and insulator; particularly, the at least one target nucleic acid sequence is a promoter.

3. The method according to any of claims 1-2, wherein the affinity tag comprises biotin and the ligand of the affinity tag comprises streptavidin.

4. The method according to any of claims 1-3 further comprising, after step (e) and before step (f), the step (e') purifying the nucleic acid sequences.

5. The method according to claim 4, with the proviso that a single purification step is carried out between steps (e) and (f).

6. The method according to any of claims 1-5 further comprising, between step (h) and step (i), the step (h') purifying the amplified sequences of length from 100 to 1000 base pairs; particularly, purifying the amplified sequences of length from 300 to 800 base pairs.

7. The method according to any of claims 1-6 further comprising, between step (f) and step (g), the step (f') end-repairing the sheared nucleic acid sequences.

8. The method according to any of claims 1-7, wherein the ligand of the affinity tag in step (g) is attached to a substrate and step (h) is carried out on the substrate

9. The method according to claim 8, wherein the substrate is a solid substrate; particularly a magnetic bead.

10. The method according to any of claims 1-9, wherein:
- the fragmenting in step (b) comprises digesting with at least one restriction endonuclease;
- the shearing in step (f) comprises sonicating; and/or
- the amplifying in step (h) comprises ligating adapters and amplifying by polymerase chain reaction (PCR).

11. The method according to any of claims 1-10, wherein the nucleic acid composition comprises chromosomal DNA.

12. The method according to claim 11, wherein the chromosomal DNA consists of the chromosomal DNA of less than 40 million cells, less than 1 million cells, less than 500.000 cells, less than 250.000 cells, less than 100.000 cells, or less than 50.000 cells.

13. The method according to any of claims 1-12, wherein steps (a) to (e) are carried out in a single reaction tube; and/or steps (g) to (h) are carried out in a single reaction tube.

14. The method according to any of claims 1-13, wherein step (i) comprises contacting the amplified nucleic acid sequences with nucleic acid probes that specifically bind to the at least one target nucleic acid sequence, and purifying the nucleic acid probes bound to the nucleic acid sequences the comprise the at least one target nucleic acid sequence or a portion of the at least one target nucleic acid sequence.

15. The method according to claim 14, wherein the nucleic acid probes are RNA probes; particularly, biotinylated RNA probes.
